(19) Europäisches Patentamt
European Patent Office
Office européen des brevets

(11) EP 4 052 711 A1

(12) # EUROPEAN PATENT APPLICATION
published in accordance with Art. 153(4) EPC

(43) Date of publication:
**07.09.2022 Bulletin 2022/36**

(21) Application number: **20882113.2**

(22) Date of filing: **26.10.2020**

(51) International Patent Classification (IPC):
***A61K 31/715*** *(2006.01)* ***A61K 31/7032*** *(2006.01)*
***A61P 25/28*** *(2006.01)* ***A61P 25/16*** *(2006.01)*
***A61P 25/04*** *(2006.01)*

(52) Cooperative Patent Classification (CPC):
**A61K 31/7032; A61K 31/715; A61P 3/10; A61P 25/04; A61P 25/06; A61P 25/16; A61P 25/28; A61P 29/00**

(86) International application number:
**PCT/CN2020/123750**

(87) International publication number:
**WO 2021/083089 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.10.2019 CN 201911057518**

(71) Applicant: **Green Valley (Shanghai) Pharmaceuticals Co., Ltd.**
**Shanghai 201203 (CN)**

(72) Inventors:
- **JIN, Yingshen**
  **Shanghai 201203 (CN)**
- **XIN, Xianliang**
  **Shanghai 201203 (CN)**
- **XIAO, Zhongping**
  **Shanghai 201203 (CN)**
- **ZHANG, Zhenqing**
  **Shanghai 201203 (CN)**
- **GENG, Meiyu**
  **Shanghai 201203 (CN)**

(74) Representative: **Murgitroyd & Company**
**Murgitroyd House**
**165-169 Scotland Street**
**Glasgow G5 8PL (GB)**

(54) **PHARMACEUTICAL COMPOSITION CONTAINING ALGINATE OLIGOSACCHARIDE DIACID**

(57) The invention relates to a pharmaceutical composition containing alginate oligosacchride diacid with formula (IV) or a pharmaceutically acceptable salt thereof, which can be used for preparing medicaments for treating Alzheimer's disease, Parkinson's disease, inflammation, pain, diabetes or vascular dementia.

HOOC
H
O
HO
OH
O
n
O
OH
m'
COOH
m
COOH
HO

formula (IV)

EP 4 052 711 A1

## Description

Technical Field

The present invention relates to a pharmaceutical composition prepared by taking alginate oligosaccharaide diacid or a pharmaceutically acceptable salt thereof as raw materials and a preparation method thereof; and belongs to the technical field of medicine.

Background

Alginic oligosaccharides have been paid extensive attention due to their potential medicinal values. Alginic oligosaccharides are usually prepared by multiple steps with alginic acid as a raw material.

In the alginic oligosaccharide molecules, there is an M segment formed of D-mannuronic acids linked by β-1,4-glucosidic bonds, a G segment formed of L-guluronic acids linked by α-1,4-glucosidic bonds, and an MG segment formed by hybridization of the two sacchorides. The structural formulae of D-mannuronic acid and L-guluronic acid are shown in Formula (I) and Formula (II) below: the algin oligosaccharide has a structural formula shown as the following formula (III):

M; β-D-mannuronic acid (I)

G; α-L- guluronic acid (II)

The structural formula of alginic oligosaccharides is shown by Formula (III) below:

(III)

The prior patent application CN201711467596.6 discloses a mannuronic acid oligosaccharide combination comprising mannuronic acid with the formula (V) or a pharmaceutically acceptable salt thereof:

(V)

wherein n is an integer selected from 1 to 9, m is selected from 0, 1 or 2, m' is selected from 0 or 1, and wherein,

the total weight of mannuronic acid wherein n=1-5 accounts for 80-95% of the total weight of the combination; the ratio of the total weight of the mannuronic diacids wherein n=1-3 to the total weight of the mannuronic diacids

wherein n=4-7 is between 1.0 and 3.5.

The mannuronic diacid oligosaccharide combination can inhibit cell damage, protect nerve cells, and increase cell survival rate. It has potential effects of preventing and treating Alzheimer disease.

In order to obtain the oligomannaric acid which has effects against Alzheimer's disease (AD) and Diabetes Mellitus in the previous patent, guluronic acid in the raw material alginic acid is removed, and the content of guluronic acid in alginic acid is usually above 30% and up to about 70%, so the actual production cost is very high in order to obtain high-purity oligomannaric acid. Therefore, the development of pharmaceutical compositions of alginate oligosacchride diacid suitable for use meets the urgent need of the industry.

Disclosure of Invention

One aspect of the invention provides a pharmaceutical composition, comprising 40-90 wt% of alginate oligosaccharide diacid or a pharmaceutically acceptable salt thereof, the structure of which is shown in formula (IV), and 8-50 wt% of a filling agent; wherein the filler is selected from starch and/or cellulose;

wherein n is an integer selected from 1 to 9, m is selected from 0, 1 or 2, and m' is selected from 0 or 1, and wherein,

the total weight of the alginate oligosaccharide diacids wherein n=1-5 accounts for more than 60% of the total weight of the combination; the total weight of guluronic acid diacid is less than 50% of the weight of the composition.

the alginic oligosaccharic acid composition of the present invention is a mixture of mannuronic acid and guluronic acid with different polymerization degrees, and its main components are oligosaccharide with a polymerization degree of 2 to 10: an M segment formed of mannuronic acids linked by $\beta$-1,4-glucosidic bonds, a G segment formed of guluronic acids linked by $\alpha$-1,4-glucosidic bonds, and an MG segment formed by hybridization of the two sacchorides. It is known that mannuronic diacids have certain pharmacological activities against Alzheimer's disease (AD) and Diabetes Mellitus. The most active saccharides are pentasaccharide to octasaccharide, especially hexasaccharide. However, the inventors find that the oligosaccharic diacid mixture of mannuronic acid and guluronic acid with a polymerization degree of 2 to 10 also has pharmacological activities against Alzheimer's disease (AD) and Diabetes Mellitus, but the premise is that the content of guluronic acid is controlled within a certain range. In other words, the alginic oligosaccharic diacid composition of the present invention can be prepared with greatly reduced production cost, which makes it easier to realize in actual production and easy to realize industrialized mass production.

In addition, alginate oligosacchride diacid is very hygroscopic and can be largely decomposed under the conditions of high temperature, strong alkali, oxidation etc. Meanwhile, since mannuronic acid and guluronic acid may be degraded during the treatment process, formic acid impurities which are harmful to the human body are generated, and thus quality control of raw materials is required to obtain an oral preparation having high safety and effectiveness. The inventors of the present application have studied and developed a novel pharmaceutical composition of alginate oligosaccharide diacid that is more stable and can be rapidly dissolved

The purity of the alginate oligosaccharide diacid or a pharmaceutically acceptable salt thereof used in the present invention is greater than 90%, preferably greater than 95%, more preferably greater than 98%. The sources of impurities are complex, and the applicant finds that one or more of the following substances may be generated in the preparation process of the alginate oligosaccharaide diacid: formic acid, glycolic acid, oxalic acid, malonic acid, hydroxymalonic acid, tartaric acid, etc. Without being bound by any theory, and without limiting the types of the impurities of the present invention, these impurities may originate from the oxidation step, or from impurities in the initial algal feedstock that are not readily detectable or from the processing of the initial algal feedstock. The inventors found that formic acid, among these impurities, is more difficult to be removed due to its small molecular weight, and has a significant influence on the stability of the formulation. Therefore, controlling the the conent of formic acid of the alginate oligosaccharide diacid or a pharmaceutically acceptable salt thereof is one of the important aspects of the present invention. Without being bound by any theory, controlling the particle size of the active ingredient in the formulation within a certain range results in a more stable formulation with less formic acid impurity.

Another aspect of the invention relates to the use of a pharmaceutical composition in the preparation of a medicament for the treatment of a disease selected from the group consisting of Alzheimer's disease, Parkinson's disease, inflammation, pain, diabetes and vascular dementia.

Yet another aspect of the invention relates to a method of treating a patient suffering from a disease selected from the group consisting of: Alzheimer's disease, Parkinson's disease, inflammation, pain, diabetes or vascular dementia, comprising administering to a patient in need thereof a pharmaceutical composition according to the invention in unit dosage form.

Yet another aspect of the invention relates to a pharmaceutical formulation comprising a pharmaceutical composition according to the invention.

Another aspect of the present invention relates to a method for preparing the pharmaceutical composition of the present invention, comprising the steps of:

(1) Mixing 40-90 wt% of alginate oligosaccharide diacid or a pharmaceutically acceptable salt thereof and 8-50 wt% of filler uniformly;
(2) The uniformly mixed material is granulated, and packed into a packaging bag quantitatively, or filled into a capsule, or tableted to obtain the pharmaceutical composition; or the uniformly mixed material obtained in step (1) is directly tableted to obtain the described pharmaceutical composition.

The formulation of the alginate oligosacchride diacid or the salt thereof has excellent preparation stability, can be quickly dissolved in an *in vivo* environment, and can ensure that the active ingredients of the alginate oligosacchride diacid can be quickly absorbed by a human body. In addition, the inventor also solved the problems arised from the preparation process of high-load alginate oligosaccharaide diacid through the composition design of the preparation, including the uniformity of the formulation, the uniform particle distribution etc.

Drawings

FIG. 1 shows the NMR spectrum of the intermediate.
FIG. 2 shows the mass spectrum of disaccharide, trisaccharide and tetrasaccharide in alginate-oligosacchride diacid API.
FIG. 3 shows a mass spectrum of pentasaccharide, hexasaccharide and heptasaccharide in alginate oligosacchride diacid API.
FIG. 4 shows a mass spectrum of octaose, nonaose and decaose in alginate oligosacchride diacid API.
FIG. 5 shows the NMR spectra of the alginate oligosaccharide diacid API.

Detailed Description

Various aspects of the present invention will be described in detail below, but the present invention is not limited to these specific embodiments. Modifications and adaptations of the present invention that come within the spirit of the following disclosure may be made by those skilled in the art and are within the scope of the present invention.

The invention provides a pharmaceutical composition, which comprises 40-90 wt% of alginate oligosaccharaide diacid or a pharmaceutically acceptable salt thereof, wherein the structure of the alginate oligosaccharaide diacid is shown in a formula (IV), and 8-50 wt% of the filler; wherein the filler is at least one selected from starch and cellulose;

HOOC, OH, HO, H, O, COOH, n, m, m'

formula (IV)

wherein n is an integer selected from 1 to 9, m is selected from 0, 1 or 2, and m' is selected from 0 or 1.

Preferably, the filler is selected from at least one of the group consisting of dry starch, corn starch, pregelatinized starch, microcrystalline cellulose, sodium carboxymethylcellulose, and croscarmellose sodium.

Preferably, the pharmaceutical composition of the present invention further comprises 0 to 10 wt% of a binder and 0.1 to 3 wt% of a lubricant; wherein the binder is at least one of starch slurry, sodium carboxymethylcellulose, hydroxypropyl cellulose, hydroxypropyl methylcellulose and polyvinylpyrrolidone; the lubricant is at least one selected from talc, magnesium stearate, silica gel micropowder, and hydrogenated vegetable oil.

Preferably, the pharmaceutical composition of the present invention comprises 60-75 wt% of alginate oligosaccharide diacid or a pharmaceutically acceptable salt thereof, 25-35 wt% of filler and 1-3 wt% of lubricant.

Preferably, the pharmaceutical composition of the present invention comprises 80-90 wt% of alginate oligosaccharide diacid or a pharmaceutically acceptable salt thereof, 8-16 wt% of filler and 1-3 wt% of lubricant.

Preferably, the particle size of the alginate oligosaccharide diacid or a pharmaceutically acceptable salt thereof (API) used in the pharmaceutical composition of the present invention is controlled such that 100wt% pass through a 60 mesh sieve, and that more than 70wt% pass through a 100 mesh sieve. Preferably, more than 80wt% pass through a 100 mesh sieve, more preferably, more than 90wt% pass through a 100 mesh sieve. Since the active ingredient has the characteristics of being easily soluble and hygroscopic, in order to make the active pharmaceutical ingredient play a better role, the inventor found that, controlling the ratio of the API particle size below 100 mesh powder and appropriately reducing the coarse raw material larger than 60 mesh will help to improve the dissolution rate of capsules and the shape of intermediate particles after granulation, respectively.

Preferably, in the pharmaceutical composition of the present invention, the total weight of the alginate oligosaccharide diacid wherein n=1 to 5 accounts for more than 60% of the total weight of the alginate oligosaccharide diacid or a pharmaceutically acceptable salt thereof; the total weight of guluronic acid diacid accounts for 0-50% of the total weight of alginate oligosaccharide diacid or a pharmaceutically acceptable salt thereof.

Preferably, in the pharmaceutical composition of the present invention, the total weight of mannuronic acid and/or guluronic acid wherein n=1 to 3 accounts for 20 to 70% of the total weight of alginate oligosaccharide diacid or a pharmaceutically acceptable salt thereof.

Preferably, in the pharmaceutical composition of the present invention, the total weight of guluronic acid diacid accounts for 5-50%, preferably 10-40%, more preferably 15-35% of the total weight of alginate oligosaccharide diacid or a pharmaceutically acceptable salt thereof.

Preferably, in the pharmaceutical composition of the present invention, the weight of the alginate oligosaccharide diacid or a pharmaceutically acceptable salt thereof with each polymerization degree accounts for the total weight of the alginate oligosaccharide diacid or a pharmaceutically acceptable salt thereof in percentage by weight as follows: 5-30% of disaccharide (n=1), 15-35% of trisaccharide (n=2), 15-35% of tetrasaccharide (n=3), 20-45% of the total content of pentasaccharide (n=4), hexasaccharide (n=5), heptasaccharide (n=6), octasaccharide (n=7), nonasaccharide (n=8) and decasaccharide (n=9).

Preferably, in the pharmaceutical composition of the present invention, the weight of the alginate oligosaccharide diacid or a pharmaceutically acceptable salt thereof with each polymerization degree accounts for the total weight of the alginate oligosaccharide diacid or a pharmaceutically acceptable salt thereof in percentage by weight as follows: 5-30% of disaccharide, 15-35% of trisaccharide, 15-35% of tetrasaccharide, 10-25% of pentasaccharide, 5-15% of hexasaccharide, 3-10% of heptasaccharide, 2-5% of octasaccharide, 1-5% of nonasaccharide and 1-5% of decasaccharide.

Preferably, in the pharmaceutical composition of the present invention, the weight of the alginate oligosaccharide diacid or a pharmaceutically acceptable salt thereof with each polymerization degree accounts for the total weight of the alginate oligosaccharide diacid or a pharmaceutically acceptable salt thereof in percentage by weight as follows: 10-29% of disaccharide, 18-32% of trisaccharide, 15-30% of tetrasaccharide, 15-20% of pentasaccharide, 5-10% of hexasaccharide, 3-5% of heptasaccharide, 2-3% of octasaccharide, 1-3% of nonasaccharide and 1-3% of decasaccharide.

In a preferred embodiment, the pharmaceutical composition according to any of the preceding claims, characterized in that the content of formic acid or a salt thereof is below 0.2%, preferably below 0.1%, more preferably below 0.05%.

Preferably, the pharmaceutical composition of the present invention comprises sodium or potassium alginate oligosaccharide diacid salt as a pharmaceutically acceptable salt.

Preferably, the pharmaceutical composition of the present invention comprises sodium alginate oligosaccharide diacid salt as a pharmaceutically acceptable salt in an amount of about 100mg to 500mg, preferably 150mg to 450 mg.

Another aspect of the invention relates to the use of a pharmaceutical composition for the preparation of a medicament for the treatment of a condition selected from the group consisting of Alzheimer’s disease, Parkinson’s disease, inflammation, pain, diabetes and vascular dementia.

Chinese patent application CN201810721327.6 discloses that oligosaccharaide diacid mixture of mannuronic acid and guluronic acid (the alginate oligosaccharaide diacid API described in the present invention) proved to have pharmacological activity against Alzheimer’s disease, Parkinson’s disease, inflammation, pain, diabetes or vascular dementia. Therefore, the pharmaceutical composition containing alginate derived oligosaccharide diacid or the salt thereof also has pharmacological activity against Alzheimer’s disease, Parkinson’s disease, inflammation, pain, diabetes or vascular dementia.

Pain mentioned in the present invention includes various pains, including but not limited to acute pain, chronic pain, neuropathic pain, postoperative pain, chronic low back pain, cluster headache, herpes neuralgia, phantom limb pain, central pain, toothache, opioid-resistant pain, visceral pain, surgical pain, bone injury pain, fatigue and pain during childbirth, pain caused by burns including sunburn, postpartum pain, migraine, angina, and genitourinary tract related pain (including cystitis), vascular pain, trigeminal neuralgia, intercostal neuralgia, surgical incision pain, chronic fasciitis pain, heel pain, muscle pain, bone pain, joint pain, cancer pain, non-cancerous pain etc.

Inflammation mentioned in the present invention includes various inflammations, including but not limited to acute inflammation, chronic inflammation, vascular inflammation, neuroinflammation, central nervous system inflammation (such as multiple sclerosis, including encephalomyelitis, etc.), peripheral nerve inflammation, Arthritis (such as osteoarthritis, sacroiliitis, etc., psoriatic arthritis, rheumatoid arthritis, rheumatoid arthritis, etc.), ankylosing spondylitis, inflammatory bowel disease (such as Crohn's disease and ulcerative colon inflammation), inflammatory diabetic ulcers, systemic lupus erythematosus, inflammatory skin diseases (such as psoriasis, atopic dermatitis, eczema), etc.

Another aspect of the invention relates to a method of treating a patient suffering from a disease selected from the group consisting of Alzheimer's disease, parkinson's disease, inflammation, pain, diabetes or vascular dementia, comprising administering to a patient in need thereof a pharmaceutical composition according to the invention in unit dosage form.

Preferably, the unit dose contains 100mg to 500mg alginate oligosaccharide diacid sodium salt, preferably 150mg to 450 mg. The unit dose may be administered to the patient once or more daily.

Another aspect of the invention relates to a pharmaceutical formulation comprising the pharmaceutical composition according to the invention.

Preferably, the pharmaceutical composition of the present invention is suitable for oral administration. The use of the pharmaceutical compositions according to the invention and preferred oral dosage forms are tablets, granules, capsules, lozenges, coated tablets and other solid oral dosage forms.

Another aspect of the present invention relates to a method for preparing the pharmaceutical composition of the present invention, comprising the steps of:

(1) Mixing 40-90 wt% of alginate derived oligosaccharide diacid or a pharmaceutically acceptable salt thereof and 8-50 wt% of filler uniformly;

(2) The uniformly mixed material is granulated, and packed into a packaging bag in a quantitative manner, or filled into a capsule, or tableted to obtain the pharmaceutical composition; or directly tableting the uniformly mixed material obtained in step (1) to obtain the pharmaceutical composition.

Preferably, 0 to 10 wt% of a binder, preferably 1 to 10 wt%, more preferably 2 to 8 wt% may also be added during the preparation. It can be added when the filler and the alginate oligosaccharaide diacid or a pharmaceutically acceptable salt thereof in the step (1) are mixed; it can also be added during the granulation in the step (2). The binder is at least one selected from the group consisting of starch slurry, sodium carboxymethylcellulose, hydroxypropyl cellulose, hydroxypropyl methylcellulose, and polyvinylpyrrolidone. Alternatively, the binder may be an aqueous solution of a lower alkanol, such as methanol, ethanol,or isopropanol.

Preferably, in the preparation method of the present invention, the granulation in step (2) is dry granulation, wet granulation or boiling granulation.

Preferably, in the preparation method of the present invention, 0.1 to 3 wt% of lubricant may be further added after the completion of the granulation in step (2).

Preferably, in the preparation method of the present invention, step (2) is followed by step (3): coating the plain tablets obtained by tableting in the step (2) to obtain the pharmaceutical composition.

Preferably, the preparation method of the invention comprises the following steps:

(1) Mixing 40-90wt% of alginate oligosaccharide diacid or a pharmaceutically acceptable salt thereof and 8-50 wt% of filler uniformly;

(2) Adding binder to the uniformly mixed material to make soft material, granulating with a sieve with an aperture of 4±0.5mm, drying at 60-70°C, and granulating with a mesh with an aperture of 1.2±0.2mm, adding lubricant, mixing uniformly, and making into capsule or tablet to thin film coat.

The pharmaceutical composition of the present invention selects a specific filler, binder or lubricant, and is combined with alginate oligosaccharide or a pharmaceutically acceptable salt thereof in a specific ratio; the composition has good stability and high disintegration and dissolution rate. For example, the dissolution rate can reach more than 90% in 30 minutes.

Advantages of the present invention are further illustrated in the following non-limiting examples. However, the specific materials and amounts thereof as well as other experimental conditions used in the examples should not be construed as limiting to the present invention. Unless otherwise specified, the parts, proportions, percentages, and the like in the present invention are all calculated by mass.

**Examples**

The measurement methods employed in the following specific examples are as follows.

Dissolution rate detection method: carbazole sulfate method

According to the determination method of dissolution rate and release method (0931 the first method in the fourth general rule of the "Chinese pharmacopoeia" 2015 edition), 900mL of acetic acid-sodium acetate buffer solution (pH4.5) was used as a dissolution medium, the rotation speed is 50 r/min, the method was operated according to the method. After 30 min, a proper amount of solution was taken out. According to carbazole sulfate method, sodium glucuronate was used as a standard substance to make a standard curve, and the content of dissolution liquid and a control solution were calculated to obtain the dissolution rate.

The determination method of the content of formic acid:

Referring to 0521 Gas Chromatography in Chinese Pharmacopoeia, "General Principles", 2015 edition.
The determination method of weight average molecular weight and molecular weight distribution:

Size exclusion chromatography-light scattering system

Chromatographic conditions: waters SEC chromatography column (Waters ACQUITY UPLC@BEH125 SEC 1.7 μm 4.6×300mm column, column temperature 25°C; the mobile phase was methanol: 80mmol/L ammonium acetate 1: 4 (V/V); the flow rate was 0.1 mL/min; detector: an eighteen angle laser detector (LS) and a refractive index detector (RI, 35 °C).
The sample to be detected was diluted with a mobile phase to prepare a solution which is 10mg/mL of alginate oligosacchride as a test solution; 20 μl of test solution was injected into a liquid chromatogram, and was analyzed to obtain a multi-angle laser scattering-differential refraction chromatogram spectrum; the data was processed by spectrum processing software (ASTRA) and the weight average molecular weight and the molecular weight distribution of the sample were obtained.

Example 1: preparation of alginate oligosacchride diacid or a pharmaceutically acceptable salt thereof (API)

Step 1): Preparation of an alginic acid oligosaccharide mixture

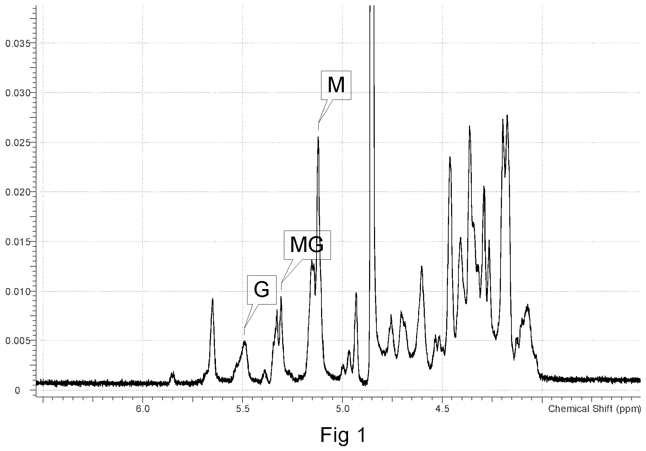

5 Kg of sodium alginicate was prepared into a solution of about 10%, and the pH was adjusted to about 3.0 by adding dilute hydrochloric acid. The solution was heated to 80°C, and stirred. It was allowed to react for 10 hr before the heating was stopped. After cooling to room temperature, the pH was adjusted to 9.0 by adding NaOH, and further adjusted to 3.2 by adding dilute hydrochloric acid. The solution was centrifuged at 5000 rpm for 10 min. The supernatant was collected, and adjusted to pH 1.0 by adding HCl. After centrifugation, the precipitate was collected, concentrated on a rotary evaporator, and dried under vaccum to give 1500 g of the intermediate.
See Figure 1 for the NMR spectrum of the intermediate. The NMR analysis was conducted as follows: sample preparation: 30 mg of the sample to be tested was weighed and dissolved in 0.5 ml D2O, and lyophilized; 0.5 ml deuterated heavy water was further added for dissolution; lyophilization was performed again; and finally, the lyophilized sample powder was dissolved with an appropriate amount of heavy water, transferred to an NMR tube, and prepared to a 100 mg/ml solution to be tested; 0.01% (w/v) deuterated TSP (trimethylsilylpropionic) sodium salt was added as an internal standard. Nuclear magnetic data acquisition and processing: 400M Fourier transform nuclear magnetic resonance instrument collected one-dimensional hydrogen spectrum at 60°C. The pulse sequence was 45° pulses, each acquisition was 4 seconds, the relaxation time was 1 second, and the accumulation was 20 times, and the spectral width was from -2 ppm to 10 ppm. After data collection, Fourier transform was used to obtain a one-dimensional hydrogen spectrum, and the TSP methyl hydrogen signal was set to 0.00 ppm.
It can be seen from Figure 1 that the intermediate contained a mannuronic acid segment (M-block, chemical shift 5.1 ppm) and a guluronic acid segment (G-block, chemical shift 5.5 ppm), as well as a chimeric segment of mannuronic acid and guluronic acid (MG-block, chemical shift 5.3 ppm). 500 g of the intermediate was weighed, and dissolved in distilled water to prepare a solution in a volume of 5 L. The solution was adjusted to pH 6.5 with NaOH, and heated in a water bath to control the reaction temperature at 75 °C. The gas flow rate at the outlet of an oxygen cylinder and the power of an ozone generator were adjusted such that ozone was fed into the reaction solution at a mass concentration flow rate of 8 g/hr. After 4 hr of reaction, the feeding of ozone was stopped, and a suitable amount of water was added to adjust the concentration of the solution to about 10%. The solution was filtered through an ultrafiltration membrane with a molecular weight cut-off of 2,000 Da to collect a retentate. The collected liquid was concentrated on a rotary evaporator and dried under vacuum to obtain 350 g API of alginate oligosaccharide diacid.

Step 2): Analysis of proportions and structures of oligosaccharides with various polymerization degrees in alginic oligosaccharic diacid API

100 mg of the above dried alginic oligosaccharic diacid API was accurately weighed, dissolved in water to a concentration of 10 mg/mL, and passed through a 0.22 um filter membrane to obtain a test sample solution. The proportions of oligosaccharides with different polymerization degrees in the composition were determined by Superdex peptide molecular exclusion chromatography (GE Co.) in combination with multi-angle laser light scattering (MALS, Wyatt Co.). The experimental conditions were as follows:

Chromatographic column: Superdex peptide 10/300G1
Mobile phase: 0.1 mol/L NaCl
Injection volume: 10 μL
Flow rate: 0.3 mL/min
Test results: from disaccharide to decasaccharide were represented by dp2-dp10, respectively, dp2 was 18%, dp3 was 24%, dp4 was 23%, dp5 was 14%, dp6 was 8%, dp7 was 7%, dp8 was 2%, dp9 was 2% and dp10 was 2%.

Step 3): LC-MS analysis of structures of oligosaccharides with various polymerization degrees in alginic oligosaccharic diacid API

Experimental conditions:

Chromatographic column: Superdex peptide 10/300G1
Mobile phase: 20% methanol + 80% 80 mmol/L $NH_4Ac$
Flow rate: 0.1 mL/ min
Column temperature: 25 °C ± 0.8 °C.
Mass spectrometry conditions: Agilent 6540 QTOF; ion source: ESI collision voltage 120 V; negative ion mode. The width of the acquired signal (m/z) was 100-1000.

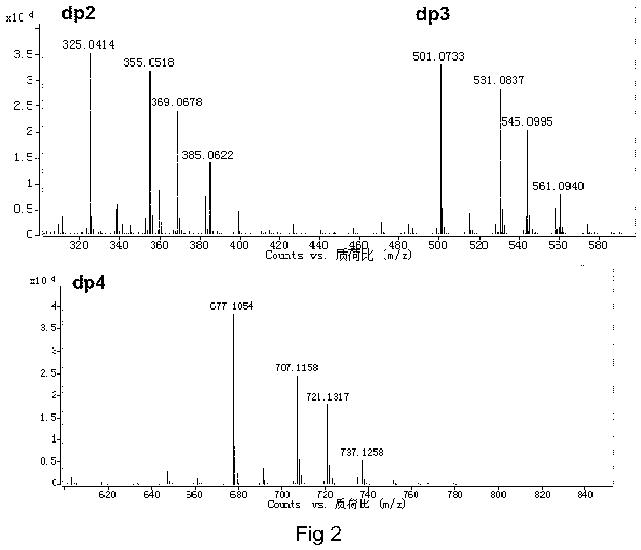

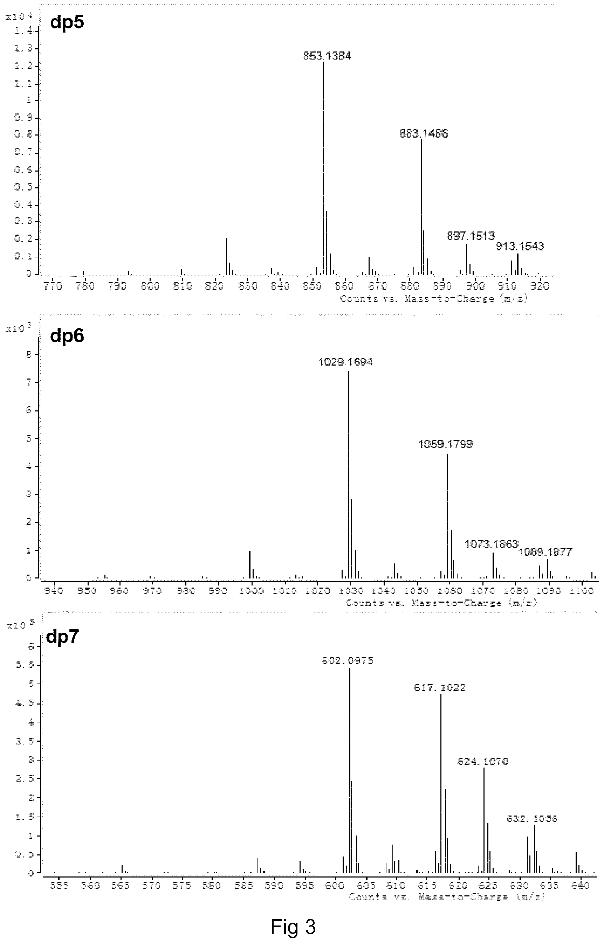

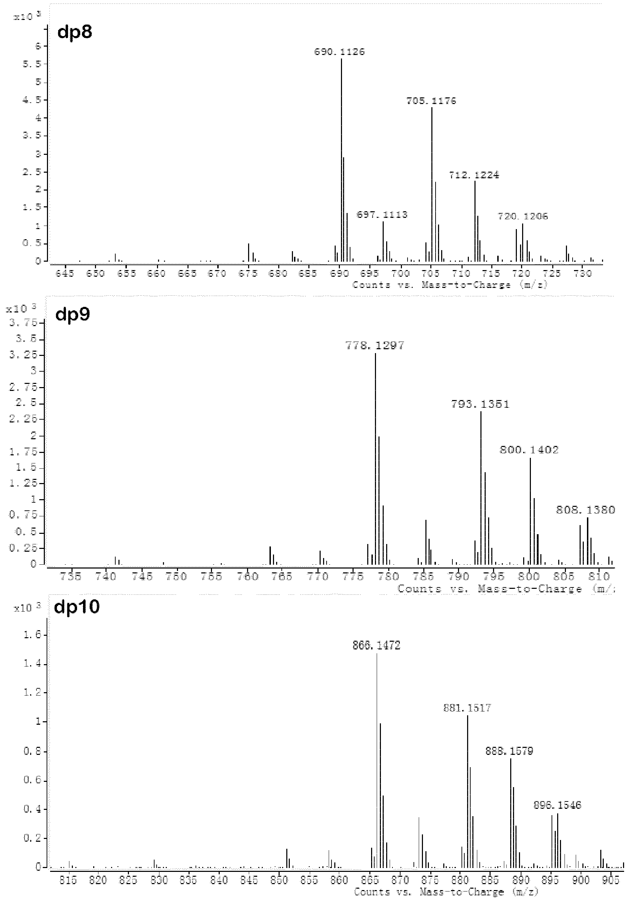

The mass spectra of oligosaccharides with various polymerization degrees are shown in Figures 2-4. Various signal peaks in the mass spectra were assigned, confirming the molecular structure of all oligosaccharides in the raw material, i.e., the structure shown in General Formula (III). See Table 1 below for the signal assignments and the structures corresponding to the signals.

Table 1: six diacid structures in oligosaccharides with different polymerization degrees in the raw material and their mass-to-charge ratios in mass spectra

| No. | Molecular Structure | Molecular Formula | Mass-to-Charge Ratio ( m/z ) | | | | | | | | |
|---|---|---|---|---|---|---|---|---|---|---|---|
| | | | n=1 $[M-1]^-$ | n=2 $[M-1]^-$ | n=3 $[M-1]^-$ | n=4 $[M-1]^-$ | n=5 $[M-1]^-$ | n=6 $[M-1]^-$ | n=7 $[M-2]^{2-}$ | n=8 $[M-2]^{2-}$ | n=9 $[M-2]^{2-}$ |
| 1 | | $(C_6H_8O_6)_nC_6H_{10}O_8$ n=1-9 | 385 | 561 | 737 | 913 | 1089 | 1265 | 720 | 808 | 896 |
| 2 | | $(C_6H_8O_6)_nC_5H_8O_7$ n=1-9 | 355 | 531 | 707 | 883 | 1059 | 1235 | 705. | 793 | 881 |
| 3 | | $(C_6H_8O_6)_nC_5H_8O_7$ n=1-9 | 355 | 531 | 707 | 883 | 1059 | 1235 | 705 | 793 | 881 |
| 4 | | $(C_5H_8O_6)_nC_4H_6O_6$ n=1-9 | 325 | 501 | 677 | 853 | 1029 | 1205 | 690 | 778 | 866 |
| 5 | | $(C_6H_8O_6)_nC_4H_6O_6$ n=1-9 | 325 | 501 | 677 | 853 | 1029 | 1205 | 690 | 778 | 866 |
| 6 | | $(C_6H_8O_6)_nC_3H_4O_5$ n=1-9 | 295 | 471 | 647 | 823 | 999 | 1175 | 675 | 763 | 851 |

It was found from the above mass spectrometric structural analysis that the mannuronic acid or the guluronic acid at the reducing end of the sugar chain in the raw material was oxidized to a saccharic acid structure (see General Formula IV for the structure), which could be a mannaric acid or guluronic acid structure comprising 6 carbon atoms (m+m'=3) with a content of about 10%~30%, or a decarboxylation product of mannaric acid or guluronic acid, i.e., a saccharic dacid comprising 5 carbons (m+m'=2) (30-50%) and a saccharide diacid with 4 carbons (m+m'=1) (30%~40%).

Step 4) NMR analysis of guluronic acid content in alginic oligosaccharic diacid raw material

Sample preparation: 50 mg of the sample to be tested was weighed, dissolved in 0.5 ml D2O, and lyophilized; 0.5 ml deuterated heavy water was added for dissolution; lyophilization was again performed; finally, the lyophilized sample powder was dissolved with an appropriate amount of heavy water, all of which was transferred to an NMR tube and prepared to a 100 mg/ml solution to be tested; and 0.01% (w/v) deuterated TSP (trimethylsilylpropionic) sodium salt was added as an internal standard.

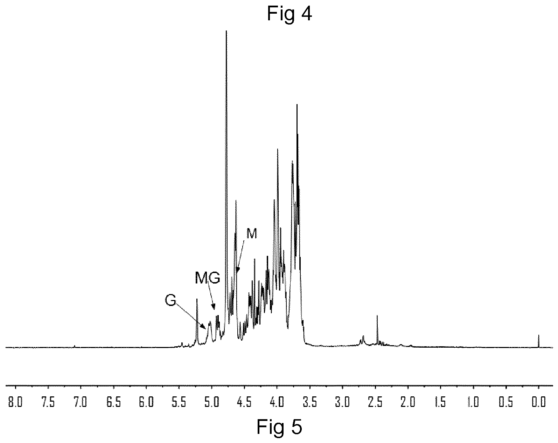

Nuclear magnetic data acquisition and processing: 400M Fourier transform nuclear magnetic resonance instrument collected one-dimensional hydrogen spectrum at room temperature. The pulse sequence was 45° pulses, each acquisition was 4 seconds, the relaxation time was 1 second, and the accumulation was 20 times, and the spectral width was from -2 ppm to 10 ppm. After data collection, Fourier transform was used to obtain a one-dimensional hydrogen spectrum, and the TSP methyl hydrogen signal was set to 0.00 ppm. The proton nuclear magnetic resonance spectrum of AI is shown in Figure 5. In Figure 5, the multiplet with a chemical shift of 4.6 ppm is the hydrogen signal at C-1 position of mannuronic acid (M), 5.0 ppm is the hydrogen signal at C-1 position of guluronic acid (G), 4.9 ppm is the C-1 hydrogen signal of chimeric segment of mannuronic acid and guluronic acid (MG). The formula for calculating the content of guluronic acid is:

$$G\% = \frac{I_{5.0} + 0.5I_{4.9}}{I_{5.0} + I_{4.6} + I_{4.9}} \times 100\%$$

In the above formula, $I_{4.6}$, $I_{5.0}$ and $I_{4.9}$ are respectively the hydrogen signal integral values at the C-1 positions of the mannuronic acid (M), the guluronic acid (G), and the chimeric segment of mannuronic acid and guluronic acid chimeric (MG). By calculation, the content of the guluronic acid in the API is 30%.

Example 2: hygroscopic of alginate oligosacchride diacid API

The alginate oligosacchride diacid API (purity 99.2%) prepared in example 1 changed from a uniformly dispersed light yellow fine powder state to yellow brown paste after being placed for 5 days in a high humidity (RH 75%, 25 °C), and the surface was smooth and glossy; it was weighed, and found to have gained more than 30% by weight.

Example 3: formulation examples

150g of the alginate oligosacchride diacid API (particle size detection: 100% passed through a 60 mesh sieve, and 80% passed through a 100 mesh sieve) prepared according to the example 1 and 55g of corn starch (commercial product, which meets the standard of Chinese pharmacopoeia, and the particle size: all pass through a 100 mesh sieve) were mixed uniformly, 85% of ethanol was used as a binder to prepare soft materials, a sieve with the aperture of 4±0.5 mm was used for granulation, dried at 60-70°C, the sieve with the aperture of 1.2±0.2 mm was used for finishing granules, 2g of magnesium stearate and 2g of talcum powder were added, the mixture was uniformly mixed and filled into capsules, and about 1000 granules were obtained.

According to the above detection method, the dissolution rate of the main active ingredients was 91.6% in 30 minutes, the content of formic acid was 0.037%, the weight average molecular weight was 925.1Da, and the molecular weight distribution was 1.051.

Example 4:

150g of the alginate oligosacchride diacid API (particle size detection: 100% passed through a 60 mesh sieve, and 80% pass through a 100 mesh sieve) prepared according to the example 1 and 65g of corn starch were mixed uniformly, 85% of ethanol was used as a binder to prepare soft materials, a sieve with the aperture of 4±0.5 mm was used for granulation, dried at 60-70°C, the sieve with the aperture of 1.2±0.2 mm was used for finishing granules, 3g of magnesium stearate and 3g of talcum powder were added, the mixture was uniformly mixed and filled into capsules,

and about 1000 granules were obtained. According to the above detection methods, the dissolution rate of the main active ingredients was 93.8% in 30 minutes, the content of formic acid was 0.041%, the weight average molecular weight was 907.9Da, and the molecular weight distribution was 1.048.

Example 5:

450g of the alginate oligosacchride diacid API (particle size detection: 100% passed through a 60 mesh sieve, and 85% pass through a 100 mesh sieve) prepared according to the example 1 and 65g of corn starch were mixed uniformly, 85% of ethanol was used as a binder to prepare soft materials, a sieve with the aperture of 4±0.5 mm was used for granulation, dried at 60-70°C, the sieve with the aperture of 1.2±0.2 mm was used for finishing granules, 2.5g of magnesium stearate and 5g of talcum powder were added, the mixture was uniformly mixed and filled into capsules, and about 1000 granules were obtained. Detection showed that the dissolution rate of the main active ingredients was 94.6% in 30 minutes, the content of formic acid was 0.039%, the weight average molecular weight was 915.2Da, and the molecular weight distribution was 1.044.

Example 6:

450g of the alginate oligosacchride diacid API (particle size detection: 100% passed through a 60 mesh sieve, and 90% pass through a 100 mesh sieve) prepared according to the example 1 and 45g of sodium carboxymethylcellulose were mixed uniformly, 85% of ethanol was used as a binder to prepare soft materials, a sieve with the aperture of 4±0.5 mm was used for granulation, dried at 60-70°C, the sieve with the aperture of 1.2±-0.2 mm was used for finishing granules, 2.5g of magnesium stearate and 5g of talcum powder were added, the mixture was uniformly mixed and tableted into a thin film coat, about 1000 granules were obtained s. Detection showed that the dissolution rate of the main active ingredients was 93.8% in 30 minutes, the content of formic acid was 0.037%, the weight average molecular weight was 918.9Da, and the molecular weight distribution was 1.046.

Example 7:

450g of the alginate oligosacchride diacid API prepared according to example 1 (particle size detection: 100% passed through a 60 mesh sieve, and 90% passed through a 100 mesh sieve) and 85g of corn starch were mixed uniformly, boiled and granulated with 5% of corn starch slurry, and a sieve with the aperture of 1.2±0.2 mm was used for finishing granules, then added 2.5g of magnesium stearate and 5g of talcum powder, mixed uniformly, and tableted into thin film coat, about 1000 granules were obtained. Detection showed that dissolution rate of the main active ingredients was 95.1% in 30 minutes, the content of formic acid was 0.032%, the weight average molecular weight was 919.3Da, and the molecular weight distribution was 1.049.

Example 8:

150g of the alginate oligosaccharide diacid API prepared according to example 1 (particle size detection: 100% passed through a 60 mesh sieve, and 90% passed through a 100 mesh sieve) and 75g of microcrystalline cellulose were mixed uniformly, 85% ethanol was used as a binder to prepare a soft material, a sieve with the aperture of 4±0.5 mm was used for granulation, dried at 60-70°C, a sieve with the aperture of 1.2±0.2 mm was used for finishing granules, 3g of magnesium stearate and 3g of talcum powder were added, the mixture was mixed uniformly, and the mixture was filled into capsules, about 1000 granules were obtained. Detection showed that the dissolution rate of the main active ingredients was 93.8% in 30 minutes, the content of formic acid was 0.041%, the weight average molecular weight was 907.9Da, and the molecular weight distribution was 1.048.

Comparative example:

450g of the alginate oligosacchride diacid API (the practicle size detection: 100% of the alginate oligosacchride diacid raw material was sieved by a 60 mesh sieve, 30% of the alginate oligosacchride diacid raw material was sieved by a 100 mesh sieve) and 20g of corn starch were mixed uniformly, 85% of ethanol was used as a binder to prepare a soft material, a sieve with the aperture of 4±0.5 mm was used for granulating, dried at 80-90°C, the sieve with the aperture of 1.2±0.2 mm was used for finishing granules, 10g of magnesium stearate and 5g of talcum powder were added, mixed uniformly, and tableted into thin film coat, about 1000 granules were obtained. Detection showed that the dissolution rate of the main active ingredients was 62.5% in 30 minutes, the content of formic acid was 0.27%, the weight average molecular weight was 905.4Da, and the molecular weight distribution was 1.046.

Example 8: accelerated stability test

The compositions prepared in examples 3-8 and comparative examples were subject to accelerated test under 40°C±2 °C /75% RH± 5% RH are given in the following table:

| | | time (month) | | | | |
|---|---|---|---|---|---|---|
| sample | Investigation items | 0 | 1 | 2 | 3 | 6 |
| Example 3 | Appearance is white to light yellow powder or granules | fit | fit | fit | fit | fit |
| | Dissolution in 30 minutes (%) | 91.6 | 91.3 | 91.5 | 91.2 | 91.3 |
| | Formic acid (%) | 0.037 | 0.037 | 0.038 | 0.039 | 0.039 |
| | Weight average molecular weight (Da) | 925.1 | 920.1 | 929.5 | 930.6 | 921.4 |
| | Molecular weight distribution | 1.051 | 1.048 | 1.052 | 1.048 | 1.047 |
| Example 4 | Appearance is white to light yellow powder or granules | fit | fit | fit | fit | fit |
| | Dissolution in 30 minutes (%) | 93.8 | 93.6 | 93.1 | 94 | 93.6 |
| | Formic acid (%) | 0.041 | 0.040 | 0.041 | 0.041 | 0.042 |
| | Weight average molecular weight (Da) | 907.9 | 910.1 | 911.9 | 900.2 | 902.4 |
| | Molecular weight distribution | 1.048 | 1.049 | 1.049 | 1.045 | 1.054 |
| Example 5 | Appearance is white to light yellow powder or granules | fit | fit | fit | fit | fit |
| | Dissolution in 30 minutes (%) | 94.6 | 94.2 | 94.6 | 94.3 | 94.5 |
| | Formic acid (%) | 0.039 | 0.039 | 0.040 | 0.041 | 0.042 |
| | Weight average molecular weight (Da) | 915.2 | 915.6 | 918.1 | 909.7 | 920 |
| | Molecular weight distribution | 1.044 | 1.048 | 1.048 | 1.043 | 1.045 |
| Example 6 | Appearance is white to light yellow powder or granules | fit | fit | fit | fit | fit |
| | Dissolution in 30 minutes (%) | 93.8 | 93.4 | 93.5 | 93.3 | 93.4 |
| | Formic acid (%) | 0.037 | 0.038 | 0.037 | 0.039 | 0.040 |
| | Weight average molecular weight (Da) | 918.9 | 915.6 | 912.6 | 916.3 | 915.6 |
| | Molecular weight distribution | 1.046 | 1.046 | 1.047 | 1.042 | 1.044 |
| Example 7 | Appearance is white to light yellow powder or granules | fit | fit | fit | fit | fit |
| | Dissolution in 30 minutes (%) | 95.1 | 95 | 95.3 | 95.1 | 95.4 |
| | Formic acid (%) | 0.032 | 0.033 | 0.034 | 0.034 | 0.035 |
| | Weight average molecular weight (Da) | 919.3 | 917.6 | 915.3 | 919.2 | 918.4 |
| | Molecular weight distribution | 1.049 | 1.05 | 1.047 | 1.049 | 1.046 |

(continued)

| | | time (month) | | | | |
|---|---|---|---|---|---|---|
| sample | Investigation items | 0 | 1 | 2 | 3 | 6 |
| Example 8 | Appearance is white to light yellow powder or granules | fit | fit | fit | fit | fit |
| | Dissolution in 30 minutes (%) | 93.8 | 93.6 | 93.6 | 93.7 | 93.6 |
| | Formic acid (%) | 0.041 | 0.041 | 0.042 | 0.043 | 0.043 |
| | Weight average molecular weight (Da) | 907.9 | 908.6 | 908.3 | 909.2 | 908.4 |
| | Molecular weight distribution | 1.048 | 1.047 | 1.049 | 1.048 | 1.049 |
| Compara tive example | Appearance is white to light yellow powder or granules | fit | fit | fit | yellow brown paste | yellow brown paste |
| | Dissolution in 30 minutes (%) | 62.5 | 62.4 | 63.1 | 62.3 | 62.5 |
| | Formic acid (%) | 0.27 | 0.29 | 0.30 | 0.32 | 0.37 |
| | Weight average molecular weight (Da) | 905.4 | 874.3 | 867.5 | 813.4 | 799.4 |
| | Molecular weight distribution | 1.046 | 1.186 | 1.25 | 1.32 | 1.57 |

**Clinical study:**

The study was a 36 week, randomized, double-blind, placebo-controlled, multicenter clinical trial with 818 patients randomized into the group and 450mg of alginate oligosacchridic diacid composition was administrated orally twice daily. The subjects were aged 50-85 years, and scored 11-26 on the MMSE scale. MRI showed that the medial temporal atrophy visual rating scale (MTA) was not greater than grade 2, the leukosis Fazekas scale was less than grade 3, no more than 2 infarcts with a diameter greater than 2 cm, no infarcts in critical brain areas, which met the clinical diagnosis criteria of mild to moderate Alzheimer's disease. The primary efficacy index was the inter-group differences compared with the placebo group of the changes from baseline after 36 weeks of treatment in the cognitive function assessment scale ADAS-cog12; the secondary efficacy index was the inter-group differences in changes from baseline in Clinician Interviewed Impression Change Scale CIBIC-plus and Activities of Daily Living ADCS-ADL Scale and Psycho-Behavioural Questionnaire NPI at 36 weeks of treatment. The safety evaluation indexes comprise adverse events, laboratory examinations, vital signs, electrocardiograms, physical examinations etc.

Clinical research results show that the alginate oligosacchride diacid composition can evidently improve cognitive dysfunction of AD patients. Compared with a placebo group, the average improvement value of the ADAS-cog12 scale is 2.54, which has extremely remarkable statistical significance ($p<0.0001$). The efficacy was particularly significant in the subgroup with a high disease severity (MMSE scale score of 11-14), with an average improvement on the ADAS-cog12 scale of 4.55 compared to the placebo group. The alginate oligosacchride diacid composition has a remarkable improvement trend (p is 0.059) on a secondary curative effect index CIBIC-plus; however, as for the ADCS-ADL tables and the NPI questionnaire, no statistical significance was observed. Compared with the placebo group, the alginate oligosaccharide diacid composition group has no significant statistical difference in the incidence of adverse events or serious adverse events, and has good safety and strong tolerance.

**Claims**

1. A pharmaceutical composition, comprising 40-90 wt% alginate oligosaccharide diacid with structure shown in formula (IV) or a pharmaceutically acceptable salt thereof, and 8-50 wt% filler; wherein the filler is selected from starch and/or cellulose;

Formula (IV)

wherein n is an integer selected from 1 to 9, m is selected from 0, 1 or 2, and m' is selected from 0 or 1.

2. The pharmaceutical composition of claim 1, wherein the filler is at least one selected from the group consisting of dry starch, corn starch, pregelatinized starch, microcrystalline cellulose, sodium carboxymethylcellulose, and croscarmellose sodium.

3. The pharmaceutical composition of claim 1, wherein the particle size of said alginate oligosaccharide diacid or a pharmaceutically acceptable salt thereof is controlled such that 100% pass through a 60 mesh sieve, and more than 70% pass through a 100 mesh sieve, preferably more than 80% of pass through the 100 mesh sieve, and more preferably more than 90% pass through the 100 mesh sieve.

4. The pharmaceutical composition of claim 1, further comprising 0 to 10 wt% of binder, preferably 1 to 10 wt%, more preferably 2 to 8 wt% of binder, and 0.1 to 3 wt% of lubricant; wherein the binder is at least one of starch, sodium carboxymethyl cellulose, hydroxypropyl methyl cellulose and polyvinylpyrrolidone; the lubricant is at least one selected from talcum powder, magnesium stearate, silica gel micropowder and hydrogenated vegetable oil.

5. The pharmaceutical composition according to any one of claims 1 to 4, wherein the total weight of the alginate oligosacchridic acids wherein n=1-5 or a pharmaceutically acceptable salt thereof is more than 60% of the total weight of the alginate oligosacchridic acids or a pharmaceutically acceptable salt thereof; the total weight of guluronic acid diacid accounts for 0-50% of the total weight of alginate oligosaccharide diacid or a pharmaceutically acceptable salt thereof.

6. The pharmaceutical composition according to any one of claims 1 to 4, wherein the total weight of mannuronic acid and/or guluronic acid wherein n=1-3 in the alginate oligosaccharide diacid accounts for 20-70% of the total weight of the alginate oligosaccharide diacid or a pharmaceutically acceptable salt thereof.

7. The pharmaceutical composition according to claim 6, wherein the total weight of the guluronic acid is between 5 and 50%, preferably between 10 and 40%, more preferably between 15 and 35% of the total weight of the alginate oligosaccharide diacid or a pharmaceutically acceptable salt thereof.

8. The pharmaceutical composition of claim 7, wherein the weight of the alginate oligosaccharide diacid or a pharmaceutically acceptable salt thereof with each polymerization degree based on the total weight of the alginate oligosaccharide diacid or a pharmaceutically acceptable salt thereof is as follows: 5-30% of disaccharide, 15-35% of trisaccharide, 15-35% of tetrasaccharide and 20-45% of total content of pentasaccharide, hexasaccharide, heptasaccharide, octasaccharide, nonasaccharide and decasaccharide.

9. The pharmaceutical composition of claim 6, wherein the weight of the alginate oligosaccharide diacid or a pharmaceutically acceptable salt thereof with each polymerization degree accounts for the following percentage of the total weight of the alginate oligosaccharide diacid or a pharmaceutically acceptable salt thereof: 5-30% of disaccharide, 15-35% of trisaccharide, 15-35% of tetrasaccharide, 10-25% of pentasaccharide, 5-15% of hexasaccharide, 3-10% of heptasaccharide, 2-5% of octasaccharide, 1-5% of nonasaccharide and 1-5% of decasaccharide.

10. The pharmaceutical composition of claim 9, wherein the weight of the alginate oligosaccharide diacid or a pharmaceutically acceptable salt thereof with each polymerization degree based on the total weight of the alginate oligosaccharide diacid or a pharmaceutically acceptable salt thereof is as follows: 10-29% of disaccharide, 18-32% of trisaccharide, 15-30% of tetrasaccharide, 15-20% of pentasaccharide, 5-10% of hexasaccharide, 3-5% of heptasaccharide, 2-3% of octasaccharide, 1-3% of nonasaccharide and 1-3% of decasaccharide.

11. The parmaceutical composition according to any of the preceding claims, wherein the content of formic acid or a

salt thereof is lower than 0.2%, preferably lower than 0.1%, more preferably lower than 0.05%.

**12.** The pharmaceutical composition of any one of claims 1-4, wherein the pharmaceutically acceptable salt is a sodium or potassium salt.

**13.** Use of a pharmaceutical composition according to any one of claims 1 to 12 in the preparation of a medicament or nutraceutical for the treatment of a disease selected from the group consisting of Alzheimer's disease, Parkinson's disease, inflammation, pain, diabetes and vascular dementia.

**14.** A method of treating a patient having a disease selected from the group consisting of Alzheimer's disease, Parkinson's disease, inflammation, pain, diabetes or vascular dementia, comprising administering to a subject in need thereof a pharmaceutical composition according to any one of claims 1-12 in unit dosage form.

**15.** A pharmaceutical formulation comprising a pharmaceutical composition according to any one of claims 1 to 12.

**16.** The pharmaceutical formulation of claim 15, wherein the formulation is administered orally.

**17.** The pharmaceutical formulation of claim 15, wherein the formulation is a tablet, a granule, or a capsule.

**18.** A process for preparing a pharmaceutical composition according to any one of claims 1 to 12, comprising the steps of:

(1) mixing 40-90 wt% of alginate oligosaccharide diacid or a pharmaceutically acceptable salt thereof and 8-50 wt% of filler uniformly;
(2) granulating the uniformly mixed materials to obtain the pharmaceutical composition; or directly tabletting the uniformly mixed material obtained in the step (1) to obtain the pharmaceutical composition;

wherein a binder is optionally added in step (1) or step (2) in an amount of 0 to 10% by weight based on the mixture obtained in step (1).

**19.** The method of claim 18, wherein the granulation in step (2) is dry granulation, wet granulation or boiling granulation.

**20.** The method of claim 19, wherein 0.1-3 wt% of lubricant is further added after the completion of the granulation in step (2).

**21.** The method according to claim 18, wherein step (2) is followed by step (3): coating the plain tablets obtained by tabletting in the step (2) to obtain the pharmaceutical composition.

**22.** The method of claim 18, comprising the steps of:

(1) mixing 40-90 wt% of alginate oligosaccharide diacid or a pharmaceutically acceptable salt thereof and 8-50 wt% of filler uniformly;
(2) adding binder into the mixed materials to make soft materials, granulating with $4\pm0.5$ mm mesh sieve, drying at 60-70°C, finishing granules with $1.2 \pm 0.2$ mm mesh sieve, adding lubricant, mixing uniformly, and filling into capsule or tableting to thin film coat.

M
MG
G
0.035
0.030
0.025
0.020
0.015
0.010
0.005
0
6.0
5.5
5.0
4.5
Chemical Shift (ppm)

Fig 1

dp2
dp3
x10 4
325.0414
355.0518
369.0678
385.0622
501.0733
531.0837
545.0995
561.0940
Counts vs. 质荷比 (m/z)
dp4
x10 4
677.1054
707.1158
721.1317
737.1258
Counts vs. 质荷比 (m/z)

Fig 2

dp5
x10⁴
853.1384
883.1486
897.1513
913.1543
Counts vs. Mass-to-Charge (m/z)
dp6
x10³
1029.1694
1059.1799
1073.1863
1089.1877
Counts vs. Mass-to-Charge (m/z)
dp7
x10³
602.0975
617.1022
624.1070
632.1056
Counts vs. Mass-to-Charge (m/z)

Fig 3

dp8
x10 3
690.1126
705.1176
712.1224
697.1113
720.1206
Counts vs. Mass-to-Charge (m/z)
dp9
x10 3
778.1297
793.1351
800.1402
808.1380
Counts vs. Mass-to-Charge (m/
dp10
x10 3
866.1472
881.1517
888.1579
896.1546
Counts vs. Mass-to-Charge (m/z)

Fig 4

M
MG
G
8.0 7.5 7.0 6.5 6.0 5.5 5.0 4.5 4.0 3.5 3.0 2.5 2.0 1.5 1.0 0.5 0.0

Fig 5

## INTERNATIONAL SEARCH REPORT

International application No.
**PCT/CN2020/123750**

**A. CLASSIFICATION OF SUBJECT MATTER**

A61K 31/715(2006.01)i； A61K 31/7032(2006.01)i； A61P 25/28(2006.01)i； A61P 25/16(2006.01)i； A61P 25/04(2006.01)i

According to International Patent Classification (IPC) or to both national classification and IPC

**B. FIELDS SEARCHED**

Minimum documentation searched (classification system followed by classification symbols)

A61K,A61P

Documentation searched other than minimum documentation to the extent that such documents are included in the fields searched

Electronic data base consulted during the international search (name of data base and, where practicable, search terms used)

DWPI, SIPOABS, CNABS, CNMED, CNTXT, CNKI, REGISTRY, CAPLUS, MEDLINE, VEN, ISI-WEB OF SCIENCE, PubMed, 褐藻胶寡糖二酸, 甘露糖醛酸, D-mannuronic acid, 古罗糖醛酸, L-guluronic acid, 痴呆, 炎症, 帕金森, 疼痛, 糖尿病, Aphrenia, aphronesia, dementia, inflammation, phlogosis, ache, odyno-, pain, soreness, diabetes, diabetes mellitus

**C. DOCUMENTS CONSIDERED TO BE RELEVANT**

| Category* | Citation of document, with indication, where appropriate, of the relevant passages | Relevant to claim No. |
|---|---|---|
| PX | CN 110652517 A (SHANGHAI GREEN VALLEY PHARMACEUTICAL CO., LTD.) 07 January 2020 (2020-01-07)<br>entire document | 1-22 |
| X | CN 108283647 A (SHANGHAI GREEN VALLEY PHARMACEUTICAL CO., LTD.) 17 July 2018 (2018-07-17)<br>claims 1-13 | 1-22 |
| Y | CN 108283647 A (SHANGHAI GREEN VALLEY PHARMACEUTICAL CO., LTD.) 17 July 2018 (2018-07-17)<br>claims 1-13 | 13-14 |
| Y | CN 106344593 A (SHANGHAI GREEN VALLEY PHARMACEUTICAL CO., LTD.) 25 January 2017 (2017-01-25)<br>claims 1-7 | 13-14（in part） |
| Y | CN 106344594 A (SHANGHAI GREEN VALLEY PHARMACEUTICAL CO., LTD.) 25 January 2017 (2017-01-25)<br>claims 1-7 | 13-14（in part） |

☑ Further documents are listed in the continuation of Box C. ☑ See patent family annex.

* Special categories of cited documents:

- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 January 2021** | **27 January 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/123750**

**C. DOCUMENTS CONSIDERED TO BE RELEVANT**

| Category* | Citation of document, with indication, where appropriate, of the relevant passages | Relevant to claim No. |
|---|---|---|
| Y | CN 106344595 A (SHANGHAI GREEN VALLEY PHARMACEUTICAL CO., LTD.) 25 January 2017 (2017-01-25)<br>claims 1-7 | 13-14 |

Form PCT/ISA/210 (second sheet) (January 2015)

**INTERNATIONAL SEARCH REPORT**

International application No.
**PCT/CN2020/123750**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

[1] Claim 14 relates to a method for treatment of a disease and thus belongs to subject matter for which no search is to be performed (PCT Rule 39.1(iv)), but a search has nevertheless been carried out for a corresponding use in preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/123750**

| Patent document cited in search report | Publication date (day/month/year) | Patent family member(s) | Publication date (day/month/year) |
|---|---|---|---|
| CN 110652517 A | 07 January 2020 | WO 2020001632 A1 | 02 January 2020 |
| | | HK 40016235 A0 | 11 September 2020 |
| CN 108283647 A | 17 July 2018 | None | |
| CN 106344593 A | 25 January 2017 | CN 106344593 B | 10 January 2020 |
| CN 106344594 A | 25 January 2017 | None | |
| CN 106344595 A | 25 January 2017 | CN 106344595 B | 19 June 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201711467596
- CN 201810721327